# EUROPÄISCHE PATENTSCHRIFT

(11) **EP 0 741 774 B1**
(45) Veröffentlichungstag und Bekanntmachung des Hinweises auf die Patenterteilung: **09.12.1998**
(21) Anmeldenummer: 95906341.3
(22) Anmeldetag: 23.01.1995
(51) Int. Cl.: C11D 3/00, C11D 1/62, C11D 3/37, C11D 1/65, A61K 7/50

(54) **DETERGENSGEMISCHE**
DETERGENT MIXTURES
MELANGES DETERGENTS

(30) Priorität: 31.01.1994 DE 4402852
(43) Veröffentlichungstag der Anmeldung: 13.11.1996
(73) Patentinhaber: Henkel Kommanditgesellschaft auf Aktien, 40191 Düsseldorf (DE)
(72) Erfinder: WILSCH-IRRGANG, Anneliese, D-45549 Sprockhövel (DE); SCHAMBIL, Fred, D-40789 Monheim (DE); VÖLKEL, Theodor, D-40699 Erkrath (DE); OSSET, Miguel, E-08015 Barcelona (ES); PI, Rafael, E-08400 Granollers (ES)
(86) Internationale Anmeldenummer: EP9500230
(87) Internationale Veröffentlichungsnummer: WO9520640

(56) Entgegenhaltungen:
- EP-A- 0 199 403
- EP-A- 0 239 910
- EP-A- 0 294 894
- EP-A- 0 309 052
- EP-A- 0 456 569
- WO-A-92/17523
- FR-A- 2 409 344

## Beschreibung

### Gebiet der Erfindung

Die Erfindung betrifft Detergensgemische, enthaltend Esterquats, schmutzablösende Polymere und Alk(en)ylsulfate.

### Stand der Technik

Kationische Tenside verfügen in Abhängigkeit ihrer Struktur über die Eigenschaft, auf negativ geladene Oberflächen beispielsweise Textilfasem oder Haare aufzuziehen. Sie bewirken dabei eine Herabsetzung der elektrostatischen Aufladung und vermitteln einen angenehmen Weichgriff. In der Praxis nutzt man diesen Effekt z. B. bei der Formulierung von Wäscheweichspülem oder Haarbehandlungsmitteln. Während noch bis vor wenigen Jahren beinahe ausschließlich quartäre Ammoniumverbindungen wie beispielsweise das Dimethyldistearylammoniumchlorid als kationische Inhaltsstoffe in Frage kamen, werden in modemen Wäscheweichspülmitteln heutzutage verstärkt quatemierte Fettsäuretriethanolaminestersalze, sogenannte "Esterquats", eingesetzt, die bei vergleichbarer Avivageleistung über eine verbesserte ökotoxikologische Verträglichkeit verfügen. Übersichten zu diesen Themen sind beispielsweise von O.Ponsati in **C.R. CED-Kongress, Barcelona, 167 (1992)** und R.Puchta in **C.R. CED-Kongress, Sitges, 59 (1993)** erschienen. Nichtsdestotrotz besteht ein Marktbedürfnis nach Avivagemitteln, die Geweben einen verbesserten Weichgriff verleihen bzw. bei dem sich der gewünschte Weichgriff mit einer niedrigeren Einsatzmenge erzielen läßt.

Es ist femer bekannt, daß öliger Schmutz viel leichter von hydrophilen Geweben wie beispielsweise Baumwolle als von hydrophobem Polyestergewebe entfernt werden kann, was eine Folge der größeren Affinität der Baumwolle gegenüber Wasser und Tensiden darstellt. Dieses unterschiedliche Verhalten erklärt sich aus dem chemischen Aufbau der Fasem: Polyesterfaser stellen Copolymere aus Terephthalsäure und Ethylenglycol dar, die über nur sehr wenige freie Hydroxyl- oder Carboxylgruppen verfügen, an denen eine Wasseranlagerung erfolgen kann. Baumwolle hingegen besteht aus einem Cellulosematerial, das umgekehrt eine Vielzahl von hydrophilen Gruppen besitzt. Ein weiterer Wunsch des Verbrauchers besteht daher nach neuen Avivagemitteln, die insbesondere Polyestergewebe in einer solchen Weise ausrüsten, daß die Verschmutzungsneigung herabgesetzt wird bzw. vorzugsweise ölige Anschmutzungen bei der nachfolgenden Wäsche wieder leicht entfernt werden können.

In diesem Zusammenhang ist die Patentschrift **US 3,712,873** zu erwähnen, aus der die Verwendung von Polyesterterpolymeren mit einem Molekulargewicht im Bereich von 1000 bis 100.000 zusammen mit quartären Ammoniumverbindungen als Gewebebehandlungsmittel bekannt ist. Die Mittel werden durch Aufsprühen oder Aufklotzen auf die Textilien appliziert und sollen die Schmutzablöseeigenschaften dieser Materialien verbessem. Wie Vergleichsversuche zeigen, wird tatsächlich eine leichte Verbesserung der Auswaschbarkeit von Ölanschmutzungen erzielt, Weichgriff und Hydrophilie jedoch tendenziell verschlechtert. Aus den Offenlegungsschriften **EP-A 0239910** und **EP-A 0309502** sind Detergenszusammensetzungen bekannt, die quartäre Ammoniumverbindungen vom Esterquat-Typ zusammen mit Terephthalsäureestem als schmutzabweisenden Polymeren enthalten. Gegenstand der **FR-A 2409344** sind Zubereitungen mit Fettsäurecholinestem und Polyethylenterephthalaten.

Die Aufgabe der Erfindung hat somit darin bestanden, neue Avivagemittel zur Verfügung zu stellen, die frei von den geschilderten Nachteilen sind.

### Beschreibung der Erfindung

Gegenstand der Erfindung sind Detergensgemische, enthaltend Esterquats der Formeln **(I), (II)** oder (III), 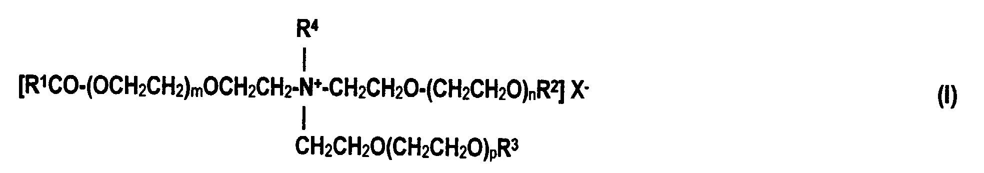 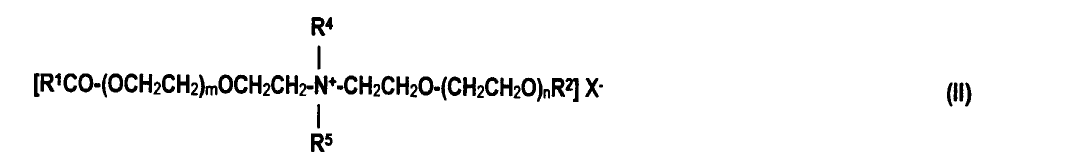 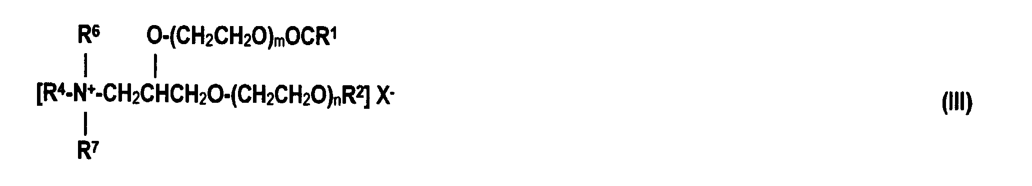 in der R¹CO für einen Acylrest mit 6 bis 22 Kohlenstoffatomen, R² und R³ unabhängig voneinander für Wasserstoff oder R¹CO, R⁴, R⁵, R⁶ und R⁷ jeweils unabhängig für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht, und schmutzabweisende Polymere mit Ethylenterephthalat- und/oder Polyethylenglycolterephthalatgruppen, die sich dadurch auszeichnen, daß sie als weitere Tenside Alkyl- und/ oder Alkenylsulfate in Mengen von 1 bis 50 Gew.-% - bezogen auf die Esterquats - enthalten.

Überraschenderweise wurde gefunden, daß der Zusatz von Alkyl- und/oder Alkenylsulfaten zu bekannten Mischungen von Esterquats und schmutzabweisenden Polymeren die Avivagewirkung der Zubereitungen in synergistischer Weise steigert. Mit dem verbesserten Weichgriff ist zugleich auch eine höhere Wiederbenetzbarkeit der Gewebe verbunden. Gleichfalls wurde gefunden, daß sowohl textile Gewebe als auch Haare, die mit den erfindungsgemäßen Detergensgemischen vorbehandelt werden, weniger stark verschmutzen und deutlich leichter von öligen Anschmutzungen befreit werden können als bei Einsatz konventioneller Weichspüler.

### Esterquats

Unter der Bezeichnung Esterquats werden im allgemeinen quatemierte Fettsäuretriethanolaminestersalze verstanden. Es handelt sich dabei um bekannte Stoffe, die man nach den einschlägigen Methoden der präparativen organischen Chemie erhalten kann. In diesem Zusammenhang sei auf die Internationale Patentanmeldung **WO 91/01295** (Henkel) verwiesen, nach der man Triethanolamin in Gegenwart von unterphosphoriger Säure mit Fettsäuren partiell verestert, Luft durchleitet und anschließend mit Dimethylsulfat oder Ethylenoxid quatemiert. Stellvertretend für den umfangreichen Stand der Technik sei an dieser Stelle auf die Druckschriften **US 3,915,867, US 4,370,272, EP-A2 0239910, EP-A2 0293955, EP-A2 0295739** und **EP-A2 0309052** verwiesen.

Typische Beispiele für Esterquats der Formel (I), die im Sinne der Erfindung Verwendung finden können, sind Produkte auf Basis von Capronsäure, Caprylsäure, Caprinsäure, Laurinsäure, Myristinsäure, Palmitinsäure, Isostearinsäure, Stearinsäure, Ölsäure, Elaidinsäure, Arachinsäure, Behensäure und Erucasäure sowie deren technische Mischungen, wie sie beispielsweise bei der Druckspaltung natürlicher Fette und Öle anfallen. Vorzugsweise werden technische C_{12/18}-Kokosfettsäuren und insbesondere teilgehärtete C_{16/18}-Talg- bzw. Palmfettsäuren sowie elaidinsäurereiche C_{16/18}-Fettsäureschnitte eingesetzt. Zur Herstellung der quatemierten Ester können die Fettsäuren und das Triethanolamin im molaren Verhältnis von 1,1 : 1 bis 3 : 1 eingesetzt werden. Im Hinblick auf die anwendungstechnischen Eigenschaften der Esterquats hat sich ein Einsatzverhältnis von 1,2 : 1 bis 2,2 : 1, vorzugsweise 1,5 : 1 bis 1,9 : 1 als besonders vorteilhaft erwiesen. Die bevorzugten Esterquats stellen technische Mischungen von Mono-, Di- und Triestern mit einem durchschnittlichen Veresterungsgrad von 1,5 bis 1,9 dar und leiten sich von technischer C_{16/18}-Talg- bzw. Palmfettsäure (lodzahl 0 bis 40) ab. Aus anwendungstechnischer Sicht haben sich quatemierte Fettsäuretriethanolaminestersalze der Formel **(I)** als besonders vorteilhaft erwiesen, in der R¹CO für einen Acylrest mit 16 bis 18 Kohlenstoffatomen, R² für R¹CO, R³ für Wasserstoff, R⁴ für eine Methylgruppe, m, n und p für 0 und X für Methylsulfat steht. Hinsichtlich der Auswahl der bevorzugten Fettsäuren und des optimalen Veresterungsgrades gelten die für **(I)** genannten Beispiele auch für die Esterquats der Formeln **(II)** und **(III)**. Üblicherweise gelangen die Esterquats in Form 50 bis 90 Gew.-%iger Mischungen mit Alkoholen in den Handel, die bei Bedarf problemlos mit Wasser verdünnt werden können.

### Schmutzablösende Polymere

Die erfindungsgemäßen Detergensgemische enthalten schmutzablösende Polymere, die vorzugsweise Ethylenterephthalat- und/oder Polyethylenglycolterephthalatgruppen aufweisen, wobei das Molverhältnis Ethylenterephthalat zu Polyethylenglycolterephthalat im Bereich von 50 : 50 bis 90 : 10 liegen kann. Das Molekulargewicht der verknüpfenden Polyethylenglycoleinheiten liegt vorzugsweise im Bereich von 750 bis 5000, d.h., der Ethoxylierungsgrad der polyethylenglycolgruppenhaltigen Polymere kann ca. 15 bis 100 betragen. Die Polymeren zeichnen sich durch ein durchschnittliches Molekulargewicht von etwa 5000 bis 200.000 aus und können eine Block-, vorzugsweise aber eine Random-Struktur aufweisen. Bevorzugte Polymere sind solche mit Molverhältnissen Ethylenterephthalat/Polyethylenglycolterephthalat von etwa 65 : 35 bis etwa 90 : 10, vorzugsweise von etwa 70 : 30 bis 80 : 20. Weiterhin bevorzugt sind solche Polymeren, die verknüpfende Polyethylenglycoleinheiten mit einem Molekulargewicht von 750 bis 5000, vorzugsweise von 1000 bis etwa 3000 und ein Molekulargewicht des Polymeren von etwa 10.000 bis etwa 50.000 aufweisen. Beispiele für handelsübliche Polymere sind die Produkte Milease® T (ICI) oder Repelotex® SRP 3 (Rhône-Poulenc). Die erfindungsgemäßen Detergensgemische können die Esterquats und die schmutzablösenden Polymere im Gewichtsverhältnis 70 : 30 bis 99,5 : 0,5, vorzugsweise 75 : 25 bis 99 : 1 enthalten. Die Angaben beziehen sich jeweils auf den Aktiv- bzw. Feststoffgehalt der Produkte.

### Alkyl- und/oder Alkenylsulfate

Die erfindungsgemäßen Detergensgemische enthalten Alkyl- und/oder Alkenylsulfate, die vorzugsweise der Formel **(IV)** folgen,

**R**^{**8**}**O-SO**_{**3**}**Y (IV)**

in der R⁸ für einen linearen oder verzweigten, aliphatischen Alkyl- und/oder Alkenylrest mit 6 bis 22 Kohlenstoffatomen und Y für ein Alkali- oder Erdalkalimetall, Ammonium, Alkylammonium, Alkanolammonium oder Glucammonium steht. Typische Beispiele für Alkylsulfate, die Sinne der Erfindung Anwendung finden können, sind die Sulfatierungsprodukte von Capronalkohol, Caprylalkohol, Caprinalkohol, 2-Ethylhexylalkohol, Laurylalkohol, Myristylalkohol, Cetylalkohol, Palmoleylalkohol, Stearylalkohol, lsostearylalkohol, Oleylalkohol, Elaidylalkohol, Petroselinylalkohol, Arachylalkohol, Gadoleylalkohol, Behenylalkohol und Erucylalkohol sowie deren technischen Gemischen, die durch Hochdruckhydrierung technischer Methylesterfraktionen oder Aldehyden aus der Roelen'schen Oxosynthese erhalten werden. Die Sulfatierungsprodukte können vorzugsweise in Form ihrer Alkalisalze, und insbesondere ihrer Natriumsalze eingesetzt werden. Besonders bevorzugt sind Alkylsulfate auf Basis von C_{16/18}-Talgfettalkoholen bzw. pflanzlicher Fettalkohole vergleichbarer C-Kettenverteilung. Die erfindungsgemäßen Detergensgemische können die Alkyl- und/oder Alkenylsulfate in Mengen von vorzugsweise 1 bis 25 Gew.-% - bezogen auf die Esterquats - enthalten. Auch diese Angaben beziehen sich wiederum auf den Aktiv- bzw. Feststoffgehalt.

### Detergensgemische

Die erfindungsgemäßen Detergensgemische stellen vorzugsweise wäßrige Produkte dar, deren Aktiv- bzw. Feststoffgehalt im Bereich verdünnter Lösungen (2 bis 10 Gew.-%) bis hin zu Konzentraten (10 bis 20 Gew.-%) und Mehrfachkonzentraten (20 bis 40 Gew.-%) liegen kann. Zu ihrer Herstellung werden entweder verdünnte Lösungen oder Konzentrate der Einzelstoffe unter Rühren und gegebenenfalls bei erhöhter Temperatur vermischt, wobei die Abfolge der Komponenten unkritisch ist. Es handelt sich dabei um einen rein mechanischen Vorgang, eine chemische Reaktion findet nicht statt. Werden Konzentrate hergestellt, so können diese in den Handel gelangen und vom Verbraucher vor Ort auf die Anwendungskonzentration verdünnt werden. Es ist femer möglich, die wasserhaltigen Detergensgemische nach bekannten Verfahren einer konventionellen Sprühtrocknung zu unterwerfen oder mit überhitztem Wasserdampf zu behandeln. Die dabei anfallenden wasserfreien Produkte zeichnen sich durch eine besonders vorteilhafte Kaltwasserlöslichkeit bzw. -dispergierbarkeit aus.

### Gewerbliche Anwendbarkeit

Die erfindungsgemäßen Detergensgemische verleihen Textilien, Fasem und Geweben, aber auch Haaren einen angenehmen Weichgriff, verbessem die Wiederbenetzbarkeit und emiedrigen die antistatische Aufladung. Mit den erfindungsgemäßen Detergensgemischen vorbehandelte Textilien, Fasern, Gewebe und Haare verschmutzen weniger und lassen sich zudem leichter von öligen Verunreinigungen befreien. Ein typisches wäßriges Textil- bzw. Faserhilfsmittel oder auch Haarbehandlungsmittel auf Basis der erfindungsgemäßen Detergensgemische enthält daher beispielsweise
(a) 2 bis 40, vorzugsweise 2 bis 10 Gew.-% Esterquats,
(b) 0,01 bis 5, vorzugsweise 0,1 bis 5 Gew.-% schmutzablösende Polymere und
(c) 0,1 bis 10 Gew.-% Alkyl- und/oder Alkenylsulfate.

Die erfindungsgemäßen Detergensgemische eignen sich zur Herstellung von oberflächenaktiven Mitteln, insbesondere Textil- bzw. Faserhilfsmitteln wie beispielsweise Avivage- und Wäscheweichspülmitteln sowie Haarbehandlungsmitteln wie beispielsweise Haarshampoos und Haarspülungen, in denen die Gemische in Mengen von 1 bis 50, vorzugsweise 3 bis 25 Gew.-% - bezogen auf die Mittel enthalten sein können.

### Hilfs- und Zusatzstoffe

Die erfindungsgemäßen Detergensgemische können weitere Hilfs- und Zusatzstoffe enthalten, deren Art und Menge vom angestrebten Einsatzzweck abhängig ist. So können die Gemische in untergeordneten Mengen weitere, mit den anderen lnhaltsstoffen kompatible Tenside enthalten. Typische Beispiele sind Fettalkoholpolyglycolethersulfate, Ethercarbonsäuren, Monoglyceridsulfate, Alkylamidobetaine oder Eiweißfettsäurekondensate.

Als Hilfs- und Zusatzstoffe, die die erfindungsgemäßen Detergensgemische enthalten können, kommen femer Emulgatoren wie etwa alkoxylierte Fettalkohole oder Sorbitanester in Betracht. Als **Überfettungsmittel** können Substanzen wie beispielsweise polyethoxylierte Lanolinderivate, Lecithinderivate und Fettsäurealkanolamide verwendet werden, wobei die letzteren gleichzeitig als Schaumstabilisatoren dienen. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, femer höhermolekulare Polyethylenglycolmono- und -diester von Fettsäuren, Polyacrylate, Polyvinylalkohol und Polyvinylpyrrolidon sowie Elektrolyte wie Kochsalz und Ammoniumchlorid. Unter **biogenen Wirkstoffen** sind beispielsweise Pflanzenextrakte, Eiweißhydrolysate und Vitaminkomplexe zu verstehen. Gebräuchliche Filmbildner sind beispielsweise Polyvinylpyrrolidon, Vinylpyrrolidon-Vinylacetat-Copolymerisate, Polymere der Acrylsäurereihe, quatemäre Cellulose- Derivate und ähnliche Verbindungen. Als **Konservierungsmittel** eignen sich beispielsweise Phenoxyethanol, Formaldehydlösung, Parabene, Pentadiol oder Sorbinsäure. Als **Perlglanzmittel** kommen beispielsweise Glycoldistearinsäureester wie Ethylenglycoldistearat, aber auch Fettsäuremonoglycolester in Betracht. Als **Farbstoffe** können die für kosmetische Zwecke geeigneten und zugelassenen Substanzen verwendet werden, wie sie beispielsweise in der Publikation "**Kosmetische Färbemittel" der Farbstoffkommission der Deutschen Forschungsgemeinschaft, veröffentlicht** **im Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt. Der Gesamtanteil der Hilfs- und Zusatzstoffe kann 1 bis 50, vorzugsweise 5 bis 40 Gew.-%-bezogen auf die Mittel - ausmachen.

### Beispiele

Durch wiederholtes Waschen gehärtetes Baumwollgewebe (Molton) wurde im Wacker-Gerät mit verschiedenen Rezepturen behandelt. Dabei galten folgende Vorgaben:

| | |
|---|---|
| Konzentration | 4 g/l |
| Flottenbeladung | 1 Teil Gewebe / 10 Teile Wasser |
| Wasserhärte | 16 °d |
| Spüldauer | 5 min |

Die Beurteilung des Weichgriffs erfolgte subjektiv durch 6 erfahrene Personen, die auf einer Skala von 0 = hart und rauh bis 6 = weich und voluminös Noten vergeben konnten. Anschließend wurde in einem Launderometer ein Polyester (veredelt)-Gewebe über 5 Zyklen mit den gleichen Weichspülerrezepturen behandelt. Dabei galten folgende Vorgaben:

| | |
|---|---|
| Weichspülerdosierung | 4 g/l |
| Flottenbelastung | 1 Teil Gewebe/10 Teile Wasser |
| Wasserhärte | 16°d |
| Spüldauer | 10 min |

Zwischen den 5 Zyklen wurden die Gewebe jeweils bei 40°C mit einem phosphatfreien Universalwaschmittel (Dixan®, Henkel KGaA, Dosierung 10 g/l, Flotte 1:10) gewaschen. Anschließend wurde auf das Gewebe 1 ml verschmutztes Motorenöl appliziert. Die mit den Weichspülerrezpturen vorbehandelten, ölverschmutzten Gewebe wurden sodann abermals gewaschen und die Fleckentfernung visuell wie folgt beurteilt: 0 = völlige Fleckentfernung; 1 = Spuren erkennbar; 2 = geringe Fleckreste; 3 = deutlich sichtbare Fleckreste; 4 = starke Fleckreste; 5 = Fleck voll erhalten. Die Ergebnisse sind in Tabelle 1 zusammengefaßt. Die Beispiele 1 und 2 sind erfindungsgemäß, die Beispiele V1 bis V9 dienen zum Vergleich. Die Prozentangaben verstehen sich als Gew.-%.

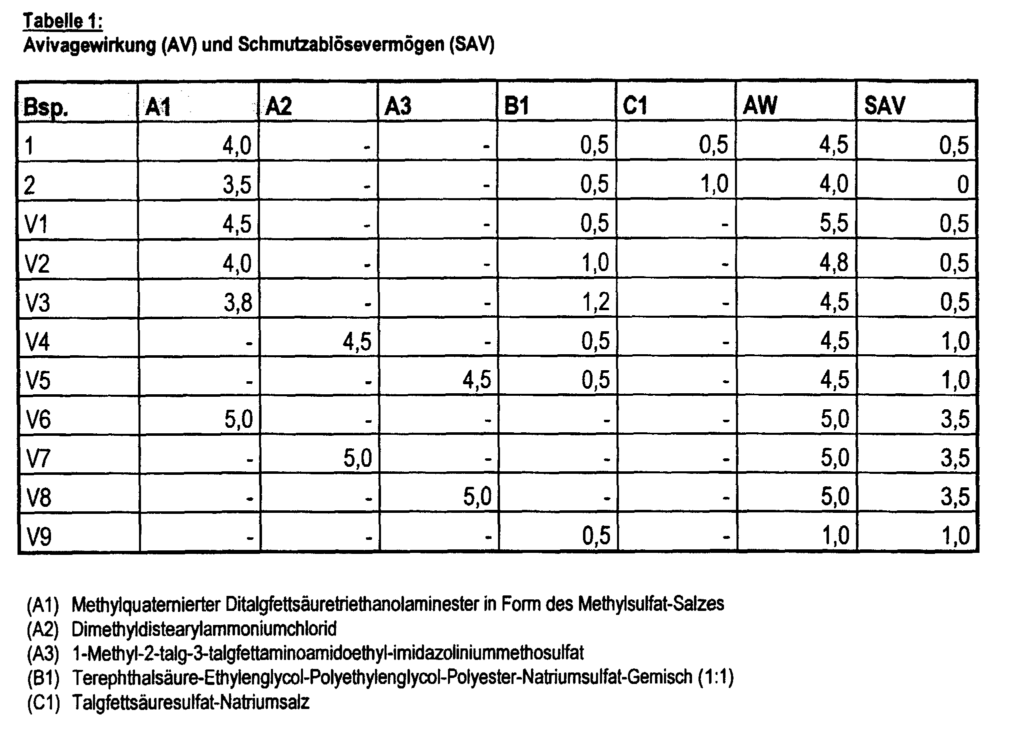

## Patentansprüche

1. Detergensgemische, enthaltend Esterquats der Formeln **(I), (II)** oder **(III)**, 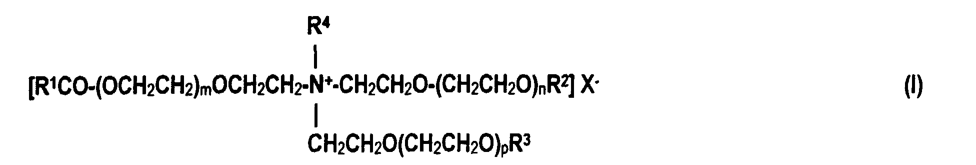 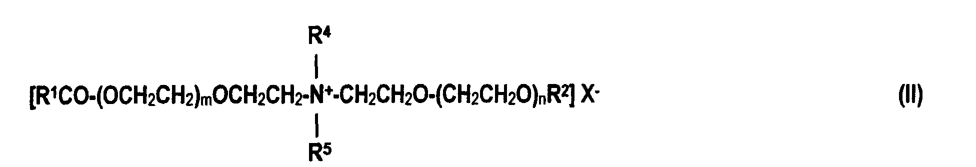 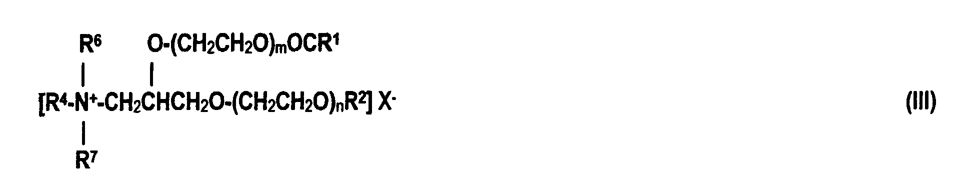 in der R¹CO für einen Acylrest mit 6 bis 22 Kohlenstoffatomen, R² und R³ unabhängig voneinander für Wasserstoff oder R¹CO, R⁴, R⁵, R⁶ und R⁷ jeweils unabhängig für einen Alkylrest mit 1 bis 4 Kohlenstoffatomen oder eine (CH₂CH₂O)_{q}H-Gruppe, m, n und p in Summe für 0 oder Zahlen von 1 bis 12, q für Zahlen von 1 bis 12 und X für Halogenid, Alkylsulfat oder Alkylphosphat steht, und schmutzabweisende Polymere mit Ethylenterephthalat- und/oder Polyethylenglycolterephthalatgruppen, **dadurch gekennzeichnet**, daß sie als weitere Tenside Alkyl- und/ oder Alkenylsulfate in Mengen von 1 bis 50 Gew.-% - bezogen auf die Esterquats - enthalten.

2. Detergensgemische nach Anspruch 1, **dadurch gekennzeichnet**, daß sie die Esterquats und die schmutzablösenden Polymere im Gewichtsverhältnis von 70 : 30 bis 99,5 : 0,5 - jeweils bezogen auf den Aktiv- bzw. Feststoffgehalt der Gemische - enthalten.

3. Detergensgemische nach den Ansprüchen 1 und 2, **dadurch gekennzeichnet**, daß sie Alkyl- und/oder Alkenylsulfate der Formel **(IV)** enthalten,
**R**^{**8**}**O-SO**_{**3**}**Y (IV)**
in der R⁸ für einen linearen oder verzweigten, aliphatischen Alkyl- und/oder Alkenylrest mit 6 bis 22 Kohlenstoffatomen und Y für ein Alkali- oder Erdalkalimetall, Ammonium, Alkylammonium, Alkanolammonium oder Glucammonium steht.

## Revendications

1. Mélanges détergents contenant des esterquats de formules **(I), (II)** ou **(III)**, 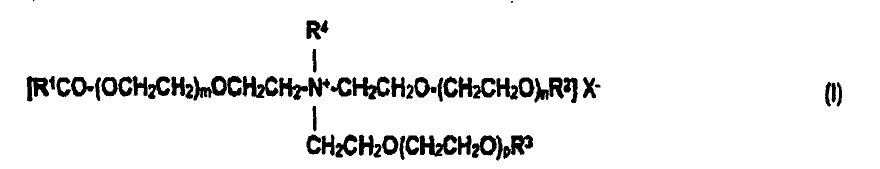 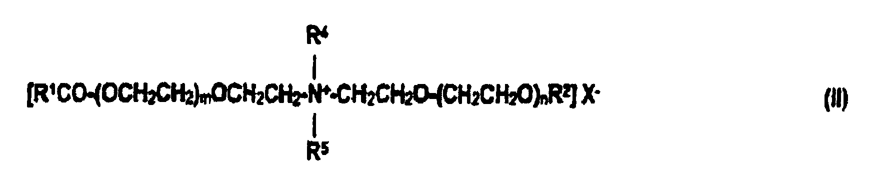 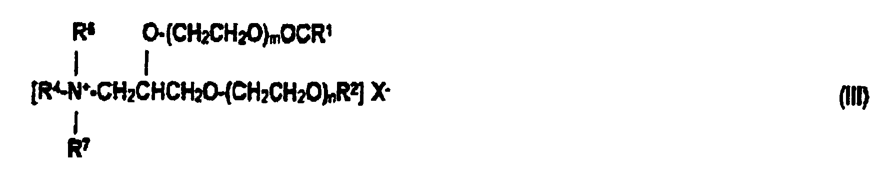 dans lesquelles R¹CO représente un radical acyle ayant de 6 à 22 atomes de carbone, R² et R³ représentent indépendamment l'un de l'autre un hydrogène ou R¹CO, R⁴, R⁵, R⁶ et R⁷ représentent chacun indépendamment un radical alkyle ayant de 1 à 4 atomes de carbone ou un groupe (CH₂CH₂O)_{q}H, la somme de m, n et p vaut 0 ou des nombres compris entre 1 et 12, q représente des nombres compris entre 1 et 12, et X représente un halogénure, un sulfate d'alkyle ou un phosphate d'alkyle, et les polymères repoussant les salissures avec des groupes téréphtalate d'éthylène et/ou téréphtalate de polyéthylène glycol,
caractérisés en ce qu'
ils contiennent comme autres agents tensioactifs des sulfates d'alkyle et/ou d'alcényle à des quantités de 1 à 50 % en poids par rapport aux esterquats.

2. Mélanges détergents selon la revendication 1,
caractérisés en ce qu'
ils contiennent les esterquats et les polymères anti taches dans un rapport pondéral de 70:30 à 99,5:0,5 - toujours par rapport à la teneur en substance active ou en solides des mélanges.

3. Mélanges détergents selon les revendications 1 et 2,
caractérisés en ce qu'
ils contiennent des sulfates d'alkyle et/ou d'alcényle de formule **(IV)**,
**R**^{**8**}**O-SO**_{**3**}**Y (IV)**
dans laquelle R⁸ représente un radical alkyle et/ou alcényle aliphatique linéaire ou ramifié ayant de 6 à 22 atomes de carbone et Y représente un métal alcalin ou alcalino-terreux, l'ammonium, un alkylammonium, alcanolammonium ou glucammonium.

## Claims

1. Detergent mixtures containing esterquats corresponding to formulae **(I), (II)** or **(III)**: 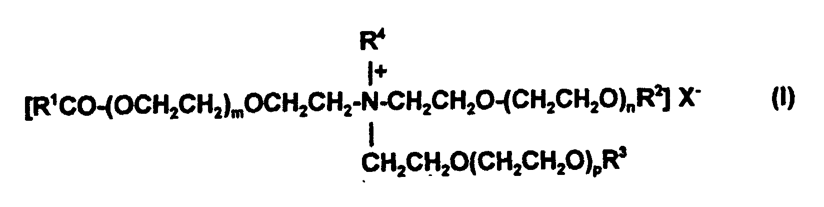 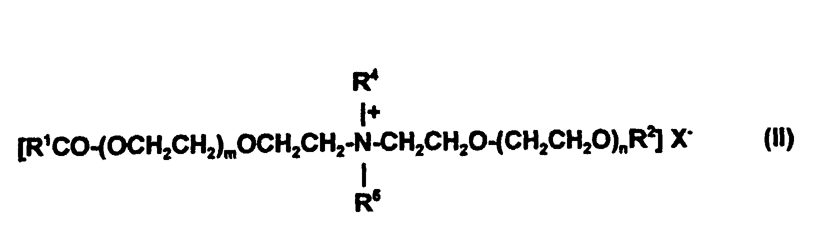 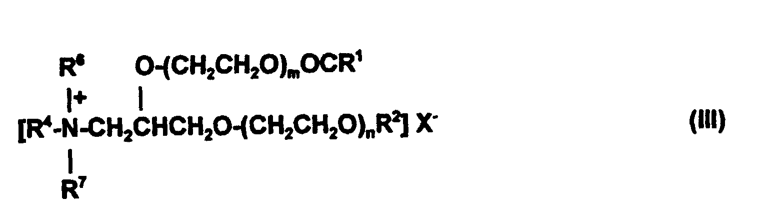 in which R¹CO is an acyl radical containing 6 to 22 carbon atoms, R² and R³ independently of one another represent hydrogen or have the same meaning as R¹CO, R⁴, R⁵, R⁶ and R⁷ independently of one another represent an alkyl group containing 1 to 4 carbon atoms or a (CH₂CH₂O)_{q}H group, m, n and p together are 0 or numbers of 1 to 12, q is a number of 1 to 12 and X stands for halide, alkyl sulfate or alkyl phosphate,
and soil-repelling polymers containing ethylene terephthalate and/or polyethylene glycol terephthalate groups, characterized in that they contain alkyl and/or alkenyl sulfates in quantities of 1 to 50% by weight, based on the esterquats, as further surfactants.

2. Detergent mixtures as claimed in claim 1, characterized in that they contain the esterquats and the soil-repelling polymers in a ratio by weight of 70:30 to 99.5:0.5, based on the active substance content or solids content of the mixtures.

3. Detergent mixtures as claimed in claims 1 and 2, characterized in that they contain alkyl and/or alkenyl sulfates corresponding to formula **(IV)**:
**R**^{**8**}**O-SO**_{**3**}**Y (IV)**
in which R⁸ is a linear or branched, aliphatic alkyl and/or alkenyl radical containing 6 to 22 carbon atoms and Y is an alkali metal or alkaline earth metal, ammonium, alkylammonium, alkanolammonium or glucammonium.
